# EUROPEAN PATENT APPLICATION

(11) **EP 4 803 581 A1**
(43) Date of publication of application: **09.09.2026**
(21) Application number: 25305313.6
(22) Date of filing: 07.03.2025
(51) Int. Cl.: C08L 101/14, C08J 3/075, C08J 3/24, A61L 27/22, A61L 27/26, A61L 27/52

(54) **WOUND DRESSING COMPRISING A REHYDRATED HYDROGEL BASED ON A GELATIN COMPONENT AND A DEGRADABLE BLOCK COPOLYMER, USEFUL IN PARTICULAR FOR TREATING CHRONIC WOUNDS**

(71) Applicant: UNIVERSITE DE MONTPELLIER, 34090 Montpellier (FR); Centre National de la Recherche Scientifique (CNRS), 75016 Paris (FR); Ecole Nationale Supérieure de Chimie de Montpellier, 34090 Montpellier (FR); Centre Hospitalier Universitaire de Nimes, 30029 Nimes Cedex 9 (FR); Skinegy, 97410 Saint Pierre (FR)
(72) Inventor: CHECKOURI, Eloïse, 97424 SAINT LEU LA RÉUNION (FR); NOTTELET, Benjamin, 34000 MONTPELLIER (FR); PINESE, Coline, 34090 MONTPELLIER (FR); SEMEDO DA MOURA, Claire, 93330 NEUILLY-SUR-MARNE (FR)
(74) Representative: Regimbeau

(57) **Abstract**

The invention relates to a dehydrated hydrogel based on:
a) a gelatin compound consisting of gelatin functionalized with at least one photoreactive group P1, and
b) a degradable branched-block copolymer comprising at least 2 degradable polymer chains, each degradable polymer chain being functionalized at its extremity with a photoreactive group P2 adapted to react in a crosslinking reaction with the photoreactive group P1.

The invention further relates to a method for preparing the dehydrated hydrogel, and to prepare a biomaterial comprising a rehydrated hydrogel obtainable or obtained by mixing the dehydrated hydrogel and an aqueous solution. The biomaterial is useful as would dressing, in particular for treating chronic wounds, such as a bedsore, an ulcer or a diabetic wound.

## Description

### FIELD OF INVENTION

The present invention relates to hydrogel-based biomaterials, useful in particular as wound dressings, advantageously for treating chronic wounds or bedsores.

### TECHNICAL BACKGROUND

Chronic wounds are a common problem in our society. They affect more than 37 million people worldwide, and their prevalence is increasing as the population ages, making them a major public health problem. There are different types of chronic wounds, such as pressure sores, lower limb ulcers and diabetic foot wounds, which are the 3 most frequently encountered types. They all present a risk of infectious complications, leading to excessive inflammation and slowing down the healing process. These complications have a major impact on patients' quality of life, ranging from the onset of severe pain to mortality in the most serious cases. The treatment of these wounds is onerous, both for the patient and for the medical profession, with a variety of people involved (nurse, attending physician, vascular surgeon, infectiologist, etc.). It involves specialized wound care, often on a daily basis, for months or even years (Abosaleh et al. Revue Francophone de Cicatrisation, 2019, 8(7)).

Therapeutic solutions have therefore been developed to promote wound healing while fighting infection. Today, there are technical dressings on the market which, unlike "conventional" dressings, are based on specific materials with the aim of incorporating biological or bioactive substances (anti-odour, pro-healing or even medicinal substances). The most common technical dressings contain silver ion precursors to help reduce the risk of antibiotic resistance and thus improve wound healing (see Su et al. Life 2021, 11, 1016). However, the use of silver entails risks of cytotoxicity, allergies and cost-effectiveness, and does not address the problem of excessive inflammation.

Various hydrogel-based solutions have been developed. Mention may be made of document CN107929804A, which discloses a modified gelatin based composite sponge, the composite sponge being based on:
a side-chain thiolated modified gelatin,
a polysaccharide comprising hyaluronic acid, chiton, chondroitin sulfate, heparin, alginic acid and a derivative thereof,
a polyethylene glycol derivative with thiol reaction activity, and
a buffer salt solution and the balance water.

But none of the solutions of the prior art is completely satisfactory. In particular, the materials of CN107929804A are not degradable as they are based on a polymer comprising polyethylene glycol derivative only, that is to say a polyether which is not bioresorbable. Thus, there is still a need for hydrogel-based wound dressings which are effective in promoting wound healing especially in the case of chronic wounds, easy to use for practitioners (in particular easy to prepare and apply on the wound), which advantageously limit inflammation reaction, may contain active ingredients useful for wound healing and avoiding infection, and which are preferably bioresorbable so as to avoid the need to change dressings, which could strip away the cellularized material resulting from ongoing cell colonization and slow healing.

### SUMMMARY OF INVENTION

The inventors have developed a dehydrated hydrogel that satisfies the above-mentioned criteria and requirements. It has been unexpectedly found that the use of a very specific combination, namely a combination of
a) a gelatin compound consisting of gelatin functionalized with at least one photoreactive group P1, and
b) a degradable block copolymer comprising at least 2 degradable polymer chains, each degradable polymer chain being functionalized at its extremity with a photoreactive group P2 adapted to react in a crosslinking reaction with the photoreactive group P1,
   allows to obtain, after a crosslinking and a dehydration step, a material that is useful in wound-dressing compositions or as biomaterial, which:
   - is effective in promoting wound healing especially in the case of chronic wounds,
   - promotes cell adhesion and colonization in particular through its porosity,
   - is easy to use for practitioners, in particular easy to prepare and apply on the wound, notably by its ability to conform to the wound shape,
   - advantageously limit inflammation reaction,
   - may contain active ingredients useful for wound healing and avoiding infection, and
   - are preferably bioresorbable so as to avoid the need to change dressings, which could strip away the cellularized material resulting from ongoing cell colonization and slow healing.

In a first aspect, the invention thus relates to a dehydrated hydrogel based on:
a) a gelatin compound consisting of a gelatin functionalized with at least one photoreactive group P1 typically selected from the group consisting of an aryl-azide group, a thiol group, and aan olefinic group such as an allyl group, a maleimide group or a (meth)acrylate group, and
b) a degradable branched-block copolymer,
   the degradable branched-block copolymer comprising a polyether central core having n arms and degradable polymer chains extending from each arm of the polyether central core, the polyether central core being star-shaped or linear, each degradable polymer chain consisting of I monomer unit(s) of a degradable polymer,
   wherein each degradable polymer chain is identical and functionalized at its extremity with a photoreactive group P2 adapted to react in a crosslinking reaction with the photoreactive group P1, the photoreactive group P2 being preferably selected from the group consisting of an aryl-azide group, a thiol group and an olefinic group such as an allyl group or a (meth)acrylate group,
   wherein said degradable branched-block copolymer is of the following formula (I): 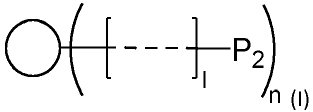
   wherein is the monomer unit of the degradable polymer constituting the degradable polymer chain,
      P₂ is the photoreactive group defined above,
      n is an integer of at least 2, and
      the polyether central core 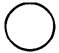 is linear, hyperbranched or star-shaped,
      when the polyether central core is linear, 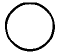 is 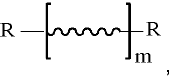 wherein R is linked to one polymer chain through a single bond or a functional group comprising a terminal function or atom selected among oxygen atom or NH group, and
      when the polyether central core is hyperbranched, is 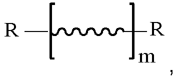 wherein R is a multivalent branched functional group comprising a number n/2 of terminal functions or atoms selected among oxygen atom or NH group, each of this terminal function being linked to one polymer chain and
      when the polyether central core is star-shaped, is 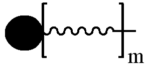 wherein is the star polyether central core,
      is the monomer unit corresponding to the polyether core, and
         m ranges from 4 to 400 and I ranges from 4 to 1500..

The dehydrated hydrogels of the invention are advantageous in that they allow adjusting the properties of the hydrogels by modifying the numbers m and I, and the gelatine compound/ degradable branched-block copolymer ratio.

Advantageously, the dehydrated hydrogel has one or more of the following features:
- the polyether of the central core of the degradable branched-block copolymer is a polyethylene glycol (PEG), a poloxamer or a poloxamine;
- the degradable branched-block copolymer has a star-shaped polyether central core and is of the following formula (II): 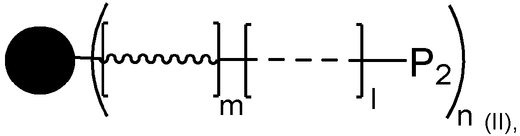 wherein 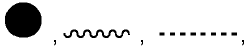 P₂, m and I are as defined herein, and n is an integer of at least 3, preferably of at least 4, more preferably n ranges from 4 to 8, even more preferably n is 4, 6 or 8.
- the degradable polymer of the degradable polymer chains of the degradable branched-block copolymer is selected from the group consisting of a polyester, a polycarbonate, and mixtures thereof, preferably a polyester;
- the polyester is selected from the group consisting of poly(lactide) (PLA), poly(ε-caprolactone) (PCL), polyhydroxybutyrate (PHB), polyhydroxybutyrate-co-hydroxyvalerate (PHBV), polyglycolic acid (PGA), poly(3-hydroxyvalerate), polydioxanone, Poly(trimethylene carbonate) (PTMC) and mixtures thereof, preferably the polyester is PLA;
- P1 and P2 are such that:
   - the photoreactive group P1 is an aryl-azide derivative, preferably an azidobenzoyl group such as 4-azidobenzoyl group, and the photoreactive group P2 is an olefinic group, such as an allyl group or a (meth)acrylate group, or
      the photoreactive group P1 is an olefinic group, such as an allyl group or a (meth)acrylate group, and the photoreactive group P2 is an aryl-azide derivative, preferably an azidobenzoyl group such as 4-azidobenzoyl group, or
   - the photoreactive groups P1 and P2 are both independently an aryl-azide derivative, preferably an azidobenzoyl group such as 4-azidobenzoyl group, or
   - the photoreactive groups P1 and P2 are both independently a (meth)acrylate group, or
   - the photoreactive group P1 is an olefinic group, such as an allyl group, a maleimide group or a (meth)acrylate group, and the photoreactive group P2 is a thiol group, or
   - the photoreactive group P1 is a thiol group and the photoreactive group P2 is an olefinic group, such as an allyl group, a maleimide group or a (meth)acrylate group;
- the weight ratio between the gelatin compound and the degradable branched-block copolymer ranges from 5 to 95 %, preferably from 25 to 75%.

In another aspect, the invention relates to a method for preparing the dehydrated hydrogel of the invention, comprising:
1) Forming an aqueous composition comprising the gelatin compound as defined herein, and the degradable branched-block copolymer as defined herein,
2) crosslinking the aqueous composition of step 1), to obtain a cured hydrogel and
3) freeze-drying the cured hydrogel of step 2).

Advantageously, the method further comprises one or more of the following features:
- the aqueous composition further comprises an initiator, preferably a photoinitiator.
- the step of forming an aqueous composition comprises heating at a temperature ranging from 15°C to 80°C, preferably from 20°C to 60°C, more preferably from 35°c to 55°C.
- the crosslinking step 2) is performed under photoreactive conditions preferably under UV irradiation.

In another aspect, the invention relates to a method for preparing a biomaterial, said method comprising:
mixing the dehydrated hydrogel of the invention or obtained by the method of the invention, with an aqueous solution optionally comprising at least one active ingredient, in particular selected from the group consisting of antibiotics, healing agents, moisturizing agents, and mixtures thereof, to obtain a hydrated hydrogel ready to be applied on a wound to be treated, typically a chronic wound, such as bedsore, leg ulcer or diabetic ulcer.

In another aspect, the invention relates to a biomaterial comprising a mixture of the dehydrated hydrogel of the invention or obtained by the method of the invention, with an aqueous solution optionally comprising at least one active ingredient, in particular selected from the group consisting of antibiotics, healing agents, moisturizing agents, and mixtures thereof, to obtain a hydrated hydrogel advantageously ready to be applied on a wound to be treated, typically a chronic wound, such as bedsore, leg ulcer or diabetic ulcer.

In another aspect, the invention relates to a use of the biomaterial of the invention as wound dressing.

In another aspect, the invention relates to the biomaterial of the invention for use for skin regeneration or for treating a wound, in particular a chronic wound, such as a bedsore, an ulcer or a diabetic wound.

### DEFINITIONS

As used herein, a "copolymer" is understood as a polymer containing several different repeated units, i.e. at least two different repeated units. A copolymer may be a random copolymer, a block copolymer or a gradient copolymer.

As used herein, a "block copolymer" is understood as a copolymer containing a sequence of different blocks, each containing only one repeated unit. A block copolymer is a single molecule, so that each block is covalently linked to the next block through a covalent bond. For instance, a block copolymer of repeated units A, B and C may have the following structure: AAAAAAAAAAABBBBBBBBBCCCCCCCCCAAAAAAAAACCCCCCCCCBBBBBBBBB.

As used herein, a "branched-block copolymer" is understood as a branched block copolymer comprising a central core and linear chains linked to the central core via a covalent bond. The number of these linear chains is of at least 2, preferably at least 3, more preferably at least 4. The core could be an atom, a molecule or a polymer (as one block copolymer). The central core is a polyether core, and may be linear or star-shaped. The linear chains extending from the core, also named "arms", consist of at least one degradable polymer.

A "linear central core" is understood as comprising at least 4 monomer units linked in a linear manner, the core being linked to linear polymer chains via functional groups comprising terminal functions or atoms such as an oxygen atom (-O-) or a -NH- (divalent) group. In this case, there are as many linear chains (arms) as terminal functions or atoms. When there are only more than 2 terminal functions (i.e. n is of 3 or more), the copolymer may be referred to as a "linear hyperbranched-block copolymer".

As used herein, a "star copolymer" or "star-shaped copolymer" is understood as a branched copolymer, wherein at least three linear chains are linked to a star-shaped central core via a covalent bond. The star-shaped central core comprises at least one atom covalently linked to at least three, preferably four, monomers, thus forming at least one star.

As used herein, "visible light" is understood as electromagnetic radiations that can be perceived by the human eye. Visible light spans the visible spectrum and is usually defined as having wavelengths ranging from 410 to 700 nm, corresponding to frequencies of 750-420 terahertz. In contrast, ultraviolet (UV) radiations have wavelengths ranging from 10 to 410 nm. As used herein, a "crosslinked" material is understood as a three-dimensional network formed by at least one polymer after being reacted with a crosslinker as defined above. A single polymer can be crosslinked provided that it has two or more arms.

When the gelatin is crosslinked with the branched-block copolymer crosslinker, it is understood that the aryl-azide functions react under visible light and/or UV irradiation, to form amine functions covalently bonding the gelatin to the branched-block copolymer. Thus, once the branched-block copolymer and the gelatin have been subjected to the crosslinking reaction, in particular under light or UV irradiation, and are crosslinked, the azide functions are not present anymore and are replaced by amine functions in the resulting crosslinked polymer.

When the gelatin is crosslinked with the branched-block copolymer crosslinker, it is understood that the (meth)acrylate functions react under visible light and/or UV irradiation, to form C-C bonds covalently bonding the gelatin and the branched-block copolymer. Thus, once the branched-block copolymer and the gelatin have been subjected to the crosslinking reaction, in particular under visible light or UV irradiation, and are crosslinked, the (meth)acrylate are not present anymore and are replaced by C-C bonds in the resulting crosslinked polymer. When the gelatin is crosslinked with the branched-block copolymer crosslinker, it is understood that the thiol functions react under visible light and/or UV irradiation, to form thio-based bonds (for example thioethers) covalently bonding the gelatin and the branched-block copolymer. Thus, once the branched-block copolymer and the gelatin have been subjected to the crosslinking reaction, in particular under visible light or UV irradiation, and are crosslinked, the thiol functions of the branched-block copolymer are not present anymore and are replaced by thio-based bonds, in particular thioether bonds, in the resulting crosslinked polymer.

As used herein, a "photoreactive group" is understood as a chemical group which undergoes chemical, structural and/or physical modifications under light activation by irradiation in the infrared, visible light and/or UV domain, typically under UV irradiation.

As used herein, a "photocrosslinkable group" is understood as a photoreactive group as defined above which is used to crosslink a polymer under light activation.

As used herein, an "aryl-azide derivative", also referred to as "aryl-azide group", is understood as a functional group comprising at least one aryl-azide function. For example, an aryl-azide derivative invention is an azidobenzoyl group.

An "aryl" refers to an aromatic hydrocarbon group comprising preferably from 5 to 12, notably from 6 to 10, carbon atoms and comprising one or more fused rings, such as, for example, a phenyl or naphtyl group. Advantageously, the aryl is a phenyl. The aryl may be unsubstituted or substituted by one or more linking group, such as -O-, -NH-, -C(O)O-, -OC(O)-, -C(O)NH-, - NHC(O)-, and the like.

As used herein, the term "olefinic group" is understood as a functional group comprising a non-aromatic double bond of formula 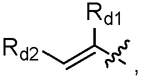 wherein 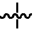 represents a link to the rest of the molecule, and R_{d1} and R_{d2} each represent independently H or a substituent such as a (C₁-C₆)alkyl group, preferably a methyl group, or taken together with the carbons bearing, form a cyclic group such as an unsaturated carbocyclyl group or an unsaturated heterocyclyl group, such as a maleimide group 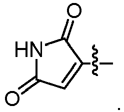 Examples of terminal double bonds include, but are not limited to, allyl group ( 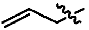 and (meth)acrylate group. As used herein, the term "(meth)acrylate group" encompasses both methacrylate ( 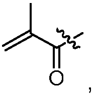 also noted MA) and acrylate ( 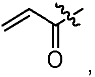 also noted A) groups.

A "(C₁-C₆)alkyl"unsaturated carbocyclyl group"

An "unsaturated heterocyclyl group" is understood as a non-aromatic, unsaturated, monocyclic or polycyclic group (comprising fused, bridged or spiro rings) comprising preferably 5 to 10, notably 5, 6, 9 or 10 atoms in the ring(s), in which one or several, notably one to four, advantageously 1, 2 or 3, ring atoms are heteroatom(s) notably selected from a sulfur atom, an oxygen atom, a nitrogen atom, or a -C(O)-group, the other ring atoms being carbon atoms. Examples of unsaturated heterocyclyl include, but are not limited to, maleimide.

As used herein, a "degradable polymer" is understood as a polymer capable of decomposition in small molecules such as water, carbon dioxide, methane, or carboxylic acids such as lactic acid, in particular by means of enzymes (enzymatic degradation, typically with micro-organism) or water (hydrolytic degradation) or any chemical reaction (for example aminolysis). "Degradable" and "biodegradable" are interchangeable in the present specification.

As used herein, a "molecular weight" refers to the number average molecular weight. The polymers of the present invention are characterized by NMR (nuclear magnetic resonance) and not by SEC (size exclusion chromatography). Of note, regarding gelatin, the molecular weight measured by NMR or SEC or SEC-MALS (Size exclusion chromatography with multi-angle static light scattering) is the "absolute" molecular weight.

As used herein, a "biomaterial" is understood as a polymeric material compatible with an animal body, including with a human body, and suitable for medical application, in particular for tissue engineering.

As used herein, "tissue engineering" refers to the technical field of repairing damaged or diseased tissues and organs. In the present invention, the organ is generally the skin, and the tissues typically include epidermis, dermis, sometimes fascia, muscle.

As used herein, PLA stands for poly(lactide). PLA₉₄ means that the PLA is constituted by 94% of L-lactic units and 6% of D-Lactic units.

As used herein, PCL stands for polycaprolactone, also named poly(ε-caprolactone).

As used herein, PHB stands for polyhydroxybutyrate.

As used herein, PH BV stands for polyhydroxybutyrate-co-hydroxyvalerate As used herein, PGA stands for polyglycolic acid.

As used herein, PLA-Pluronic^{®}-PLA stands for the copolymer comprising PLA unit and poloxamer unit and having the following structure:

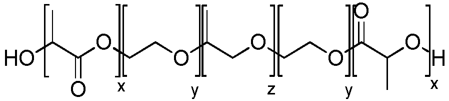

This copolymer is also referred to as PLA₅₀-Pluronic^{®}-PLA₅₀ or PLA₅₀PLU.

As used herein, "PEG" stands for polyethylene glycol. PEG is also known as polyethylene oxide (PEO) or polyoxyethylene (POE). The structure of a linear PEG is commonly expressed as H-(O-CH₂-CH₂)ₚ-OH with p being an integer of 2 or more. The term "(PEG)_{n_arm}" means that the PEG central core provides n arms in the star-shaped or hyperbranched copolymer, each arm being substituted with m unit(s) of PEG monomer.

As used herein, the term "pharmaceutically acceptable" is intended to mean what is useful to the preparation of a pharmaceutical composition, and what is generally safe and non-toxic, for a pharmaceutical use.

As used herein, a "dehydrated material" is understood as a material comprising at most 15 wt% of water, preferably at most 10 wt%, relative to the total weight of the material.

The water content of a material, in particular of the dehydrated hydrogel, may be measured by loss of mass upon desiccating, Karl-Fischer method, or thermogravimetric measurement.

### DETAILED DESCRIPTION

### I. Method for preparing a dehydrated hydrogel

The invention provides a method for preparing the dehydrated hydrogel of the invention, comprising:
1) Forming an aqueous composition comprising a gelatin compound and a degradable branched-block copolymer,
2) crosslinking the aqueous composition of step 1), to obtain a cured hydrogel, and
3) freeze-drying the cured hydrogel of step 2).

As used herein, a "hydrogel" is understood as a porous material in the form of a gel. The preparation of a gel involves a sol-gel transition step *i.e.* the modification of a suspension of solid particles, the sol, to a gelatinous material of solid appearance, the gel. In the case of hydrogels, the solvent of the sol is an aqueous solution, such as water or biological fluids. Generally, the solid phase is a water-insoluble three-dimensional network of polymers.

### Forming step 1)

Forming step 1) may be considered as a step of providing an aqueous composition comprising a gelatin compound and a degradable branched-block copolymer.

The aqueous composition comprises a solvent including at least 10 wt%, preferably at least 50 wt%, of water. In some embodiments, the aqueous composition comprises a pharmaceutically acceptable co-solvent such as a pharmaceutically acceptable lower alcohol, preferably ethanol. In other embodiments, the solvent of the aqueous solution consists of water.

Advantageously, the pH of the aqueous composition ranges from 4.5 to 8.5, preferentially from 5 to 7.5, advantageously from 6 to 7. In particular, the aqueous composition may be buffered, *i.e.* it may also comprise a buffer chosen from common buffers for the pH range 5 to 8, preferentially selected from the group consisting of acetate, lactate, tartrate, malate, maleate, succinate, ascorbate, carbonate, phosphate, Tris (Tris(hydroxymethyl)aminomethane), HEPES (2-[4-(2-hydroxyethyl)-1-piperazine]ethanesulfonic acid), MES (2-morpholinoethanesulfonic acid) and mixtures thereof, preferentially acetate, phosphate, Tris, lactate, tartrate, carbonate; MES or mixtures thereof, more preferably the phosphate or acetate buffered solutions.

The aqueous composition may further comprise additives such as antioxidants, salts, and/or stabilizers. Exemplary formulations may be found in the general literature, including Remington's for Pharmaceutical Science 18th Edition (1990), Mack. Pub. For instance, sterile aqueous or saline solutions can be prepared with galenic adjuvants (lactose, methylcellulose, mannitol), and/or surfactants (lecithins, Tween^{®} or similar products).

Preferred salts include sodium chloride.

Optionally, the aqueous solution may comprise at least one active ingredient, in particular as defined below.

The aqueous composition typically comprises a photoinitiator. Photoinitiators are well known in the art.

The aqueous composition of step 1) preferably comprises 5 wt% or less, such as 2 wt% or less, in particular from 0.1 wt% to 1 wt% of photoinitator, relative to the total weight of gelatin compound and degradable branched-block copolymer.

Advantageously, the initiator, especially the photoinitiator, is water-soluble, in particular at a temperature of between 20°C and 80°C.

Preferably, especially when P1 and/or P2 comprises an olefinic group, the photoinitator is a type I photoinitiator, such as an Irgacure^{®} photoinitiator. Such photoinitators include 2-Hydroxy-1-{4-[4-(2-hydroxy-2-methyl-propionyl)-benzyl]-phenyl}-2-methyl-propan-1-one (Irgacure 127), 2-Dimethylamino-2-(4-methyl-benzyl)-1-(4-morpholin-4-yl-phenyl)- butan-1-one (Irgacure 379), 2-Hydroxy-4'-(2-hydroxyethoxy)-2-methylpropiophenone (Irgacure 500), Phenyl bis (2,4,6-trimethylbenzoyl)phosphine oxide (BAPO ou irgagure 819), 2,2'-azobis[2-methyl-n-(2-hydroxyethyl)propionamide] (VA-086), Dimethyl(2,4,6-trimethylbenzoyl)phosphine oxide (TPO), lithium phenyl(2,4,6-trimethylbenzoyl)phosphinate (LAP), and Ethyl (2,4,6-trimethylbenzoyl)phenylphosphinate (TPO-L).

Typically, when P1 and/or P2 comprises an aryl-azide derivative, the aqueous composition is free of photoinitiator, as the aryl-azide derivative acts as a photoinitator.

In some embodiments, the aqueous composition consists of:
- the gelatin compound as defined herein,
- the degradable branched-block copolymer as defined herein,
- the aqueous solvent, and
- optionally a photoinitiator, an active ingredient, and/or additives selected from the group consisting of buffer agent, antioxidants, osmolality regulating agents, and/or stabilizers. Advantageously, the aqueous composition consists of:
- the gelatin compound as defined herein,
- the degradable branched-block copolymer as defined herein,
- the aqueous solvent, and
- a photoinitiator, and
- optionally an active ingredient, and/or additives selected from the group consisting of buffer agent, antioxidants, osmolality regulating agents, and/or stabilizers.

To favor solubilization of the components, in particular of the gelatin compound and the degradable branched-block copolymer, the step 1) of forming the aqueous composition is preferably carried out under stirring, and typically involves heating at a temperature ranging from 15°C to 80°C, preferably from 20°C to 60°C, more preferably from 35°c to 55°C.

In some embodiments, the gelatin compound and the degradable branched-block copolymer are added to the solvent to form a mixture, which then optionally stirred and/or heated to a temperature ranging from 15°C to 80°C, preferably from 20°C to 60°C, more preferably from 35°c to 55°C. Optionally, additives may be added together with the gelatin compound and the degradable branched-block copolymer, or after the gelatin compound and the degradable branched-block copolymer have been solubilized.

In some embodiments, the gelatin compound and the degradable branched-block copolymer are each solubilized separately in a solvent to form separate solutions (also called stock solutions), and the degradable branched-block copolymer solution is added to the gelatin solution (or the gelatin solution is added to the degradable branched-block copolymer solution), optionally under stirring, to form an aqueous mixture, which is then preferably heated to a temperature ranging from 15°C to 80°C, preferably from 20°C to 60°C, more preferably from 35°C to 55°C. Optionally, additives may be added to the gelatin compound solution and/or the degradable branched-block copolymer solution, and/or to the aqueous mixture or composition. Preferably, the weight ratio between the gelatin compound and the degradable branched-block copolymer in the aqueous composition ranges from 5 to 95 %, preferably from 25 to 75%. The gelatin compound consists of a gelatin functionalized with at least one photoreactive group P1 typically selected from the group consisting of an aryl-azide group, an olefinic group (such as an allyl group, a maleimide or a (meth)acrylate group), and a thiol group. The gelatin compound may be in particular as described herein under section "II. Dehydrated hydrogel". The degradable branched-block copolymer comprises a polyether central core having n arms and degradable polymer chains extending from each arm of the polyether central core, the polyether central core being star-shaped or linear, each degradable polymer chain consisting of I monomer unit(s) of a degradable polymer,
wherein each degradable polymer chain is identical and functionalized at its extremity with a photoreactive group P2 adapted to react in a crosslinking reaction with the photoreactive group P1, the photoreactive group P2 being preferably selected from the group consisting of an aryl-azide group, an olefinic group (such as an allyl group, a maleimide or a (meth)acrylate) group, and a thiol group,
wherein the degradable branched-block copolymer is of formula (I) as defined herein.

The degradable branched-block copolymer may be in particular as described herein under section "II. Dehydrated hydrogel".

In some embodiments, P1 and P2 are identical. In these embodiments, P1 and P2 are preferably both an aryl-azide derivative - preferably an azidobenzoyl group such as 4-azidobenzoyl group (Bz-N₃) - or a (meth)acrylate group.

In some embodiments wherein P1 or P2 is an aryl-azide derivative - preferably an azidobenzoyl group such as 4-azidobenzoyl group (Bz-N₃) - then the other photoreactive groupe (respectively P2 or P1) may be a single C-H bond. Preferably, in those embodiments, P2 is an aryl-azide derivative and P1 is a single C-H bond.

In some embodiments, one of P1 and P2 is a thiol group. In these embodiments, the other photoreactive group (P2 or P1 respectively) is an olefinic group, such as an allyl group or a (meth)acrylate group. In these embodiments, the crosslinking reaction is a thiol-ene reaction. In other words, advantageously,
- the photoreactive group P1 is an aryl-azide derivative, preferably an azidobenzoyl group such as 4-azidobenzoyl group, and the photoreactive group P2 is an olefinic group, such as an allyl group or a (meth)acrylate group, or
   the photoreactive group P1 is an olefinic group, such as an allyl group or a (meth)acrylate group, and the photoreactive group P2 is an aryl-azide derivative, preferably an azidobenzoyl group such as 4-azidobenzoyl group, or
- the photoreactive groups P1 and P2 are both independently an aryl-azide derivative, preferably an azidobenzoyl group such as 4-azidobenzoyl group, or
- the photoreactive groups P1 and P2 are both independently a (meth)acrylate group, or
- the photoreactive group P1 is an olefinic group, such as an allyl group, a maleimide group or a (meth)acrylate group, and the photoreactive group P2 is a thiol group, or the photoreactive group P1 is a thiol group and the photoreactive group P2 is an olefinic group, such as an allyl group, a maleimide group or a (meth)acrylate group.

### Crosslinking step 2)

The crosslinking step involves chemical reaction(s) providing a three-dimensional network via the covalently bonding of macromolecular chains. Crosslinking may be initiated by heat, pressure, change in pH, radiation and may require the use of a crosslinking agent (also called initiator).

Initiation by heat may be particularly used when the crosslinking reaction involves click-chemistry (*i.e.* one of P1 and P2 is an aryl-azide derivative, while the other is an olefinic group), or a thiol-ene reaction (*i.e.* one of P1 and P2 is a thiol, while the other is an olefinic group). Preferably, the crosslinking step 2) is carried out under photoreactive conditions, *i.e.* under irradiation in the visible light and/or UV domain, typically under UV irradiation, to promote photo-crosslinking defined as the photo-induced formation of a covalent bond between at least two macromolecular chains.

Photo-crosslinking is typically performed in the presence of a photoinitiator. The person of skill in the art will choose a photoinitiator which is compatible and suited to the reactive groups P1 and P2. The photoinitiator is especially as defined herein, preferably a type I photoinitiator, such as an Irgacure^{®} photoinitiator.

When P1 and/or P2 comprises an aryl-azide derivative, the crosslinking step 2) is preferably carried out under irradiation at a wavelength ranging from 100 to 280 nm (UV C domain).

When P1 and/or P2 comprises a terminal double, the crosslinking step 2) is preferably carried out under irradiation at a wavelength ranging from 315 to 400 nm (UV A domain).

The irradiation is typically carried out for a predetermined time, which the person of skill in the art will know how to choose based on preliminary experiments. Preferably, the irradiation is carried out for 10 minutes or less, such as 8 minutes or less. Advantageously, irradiation is carried out for at least 30 seconds, preferably at least 1 minute, more preferably at least 2 minutes.

The irradiation power flux density may range from 30 mW/cm² to 200 mW/cm².

In some embodiments, the crosslinking step is performed in a tank comprising a cooling system, allowing to limit heating or maintain the inner temperature of the reaction mixture under a predetermined temperature value, so as to avoid evaporation of the solvent and/or undesired side reaction. The predetermined temperature value generally ranges from 25 to 80°C, such as from 35 to 75 °C.

Advantageously, the only reactants in the crosslinking step 2) are the gelatin compound a) and the degradable branched-block copolymer b), and optionally an initiator such as a photoinitiator, the other components of the aqueous composition, *i.e.* water and the optional components (namely the cosolvent, the additives, and the active ingredient(s)) being inert or unreactive in the crosslinking step.

### Freeze-drying step 3)

The freeze-drying step 3) allows to dehydrate the cured hydrogel obtained in step b), thus creating porosity within it. The dehydrated cured hydrogel thus obtained may thus be regarded as a polymeric sponge.

During the freeze-drying step 3), the temperature is lowered to a target temperature T_{d} below 0°C. Preferably, the target temperature T_{d} is of -20°C or less, more preferably of -30°C or less, even more preferably of -45°C or less, such as -50°C.

Advantageously, the freeze-drying step 3) is carried out at a pressure P_{d} below the atmospheric pressure, *i.e.* at a pressure P_{d} below 1 bar, preferably at a pressure P_{d} of 0.01 bar or less, more preferably at a pressure P_{d} of 0.001 bar or less, even more preferably at a pressure P_{d} of 0.0001 bar or less, and preferably P_{d} is of 0.00001 bar or more.

During step c), the reaction mixture is typically maintained at the target temperature T_{d} and at the pressure P_{d} during at least 1h, preferably during at least 6h, such as during at least 10h. Preferably, the reaction mixture is maintained at the target temperature T_{d} and at the pressure P_{d} during at most 24h, and more preferably during at most 18h.

Advantageously, at the end of the lyophilizing step 3), the cured hydrogel is dehydrated. Therefore, the process of the invention yields a dehydrated cured hydrogel.

### II. Dehydrated hydrogel

The invention also provides a dehydrated hydrogel based on:
a) a gelatin compound consisting of a gelatin functionalized with at least one photoreactive group P1 typically selected from the group consisting of an aryl-azide group, a thiol group, and an olefinic group (such as an allyl group, a maleimide or a (meth)acrylate group), and
b) a degradable branched-block copolymer,
   the degradable branched-block copolymer comprising a polyether central core having n arms
   and degradable polymer chains extending from each arm of the polyether central core, the polyether central core being star-shaped or linear, each degradable polymer chain consisting of I monomer unit(s) of a degradable polymer,
   wherein each degradable polymer chain is identical and functionalized at its extremity with a photoreactive group P2 adapted to react in a crosslinking reaction with the photoreactive group P1, the photoreactive group P2 being preferably selected from the group consisting of an aryl-azide group, an olefinic group (such as an allyl group, a maleimide or a (meth)acrylate group), and a thiol group,
   wherein the degradable branched-block copolymer is of the following formula (I): 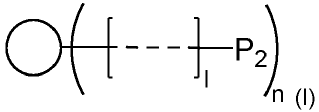
   wherein is the monomer unit of the degradable polymer constituting the degradable polymer chain,
      P₂ is the photoreactive group defined herein,
      n is an integer of at least 2, preferably of at least 3, more preferably of at least 4, even more preferably between 4 and 8, and
      the polyether central core 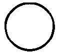 is linear, hyperbranched or star-shaped,
      when the polyether central core is linear, is 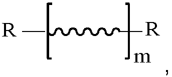 wherein R is linked to one polymer chain through a single bond or a functional group comprising a terminal function or atom selected among oxygen atom or NH group, and
      when the polyether central core is hyperbranched, is 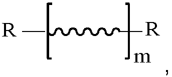 wherein R is a multivalent branched functional group comprising a number n/2 of terminal functions or atoms selected among oxygen atom or NH group, each of this terminal function being linked to one polymer chain and
      when the polyether central core is star-shaped, is 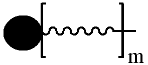 wherein is the star polyether central core,
      is the monomer unit corresponding to the polyether core, and
         m ranges from 4 to 400 and I ranges from 4 to 1500.

As used herein, a "hydrogel based on" refers to a hydrogel comprising the mixture and/or the product of a reaction of at least the gelatin compound a) and the degradable branched-block copolymer b), which are intended to react at least in part with one another, and optionally with their close chemical environment, during the crosslinking step. Preferably, a hydrogel based on the gelatin compound a) and the degradable branched-block copolymer b) consists of the product of the reaction between the base constituents a) and b), and optionally an initiator such as a photoinitiator, the reaction being the crosslinking of the crosslinking step.

The dehydrated hydrogel is thus obtainable or obtained by the method of the invention.

Advantageously, the dehydrated hydrogel is porous, with pores present inside the material, in particular at the core of the dehydrated hydrogel. The porosity is advantageously adapted to promote cell adhesion and colonization. Typically, the porosity of the dehydrated hydrogel ranges from 50% to 95%, typically from 70% et 90%, as measured by SEM or pycnometry. Preferably, the dehydrated hydrogel has a pore size ranging from 10 to 500 µm, preferably 50 to 300 µm. The pore size may for instance be measured using SEM or by tomography.

### Gelatin

The gelatin compound consists of a gelatin functionalized with at least one photoreactive group P1.

Gelatin is an irreversibly hydrolyzed form of collagen (generally of animal source), wherein the hydrolysis reduces the collagen protein fibrils into smaller peptides. Depending on the physical and chemical methods of denaturation, the molecular weight of the peptides may fall within a broad range.

Many processes for converting collagen into gelatin have been described. They generally comprise the following steps:
- Pretreatment(s) of the collagen to remove impurities that may have negative effects on the physicochemical properties of the final gelatin product and denature at least in part the collagen to facilitate hydrolysis, thereby obtaining a pre-treated collagen,
- Hydrolysis of the pre-treated collagen into gelatin,
- Extraction of gelatin from the hydrolysis mixture, which usually is carried out using hot water or dilute acid solutions as a multistage process,
- refining and/or recovering treatment step(s) including filtration, clarification, evaporation, sterilization, drying, rutting, grinding, and/or sifting, to remove the water from the gelatin solution, to blend the gelatin extracted, and to obtain dried, blended, ground final product.

Gelatin obtained from acid-treated raw material is called type-A gelatin while gelatin obtained from alkali-treated raw material is referred to as type-B gelatin.

Gelatin A and/or Gelatin B, in particular Gelatin A, may then be subjected to a functionalization step, to provide the gelatin compound functionalized with at least one photoreactive group P1. Functionalization steps are well known in the art. For instance, when the photoreactive group P1 is a terminal double such as a (meth)acrylate group, the reaction conditions and protocols described in Shirahama et al. (Sci Rep. 2016;6(1):31036) or in Lee et al. (RSC Adv. 2015;5(128):106094-7) may be used.

Advantageously, the weight ratio between the gelatin compound a) and the degradable branched-block copolymer b) ranges from 5 to 95 %, preferably from 25 to 75%.

### Degradable branched-block copolymer

The degradable branched-clock copolymer A may be obtained following the methods described in WO2023/ 047060 or in WO2020144236A1.

The numbers n, m and I in the branched-block copolymer are advantageously chosen so as to obtain the optimal compromise between a high molecular weight of the branched-block copolymer itself and a high reactivity of the copolymer chain ends.

Advantageously, a number of arms "n" of at least 4 allows ensuring a high crosslinking yield between the branched-block copolymer and the gelatin, despite the high molecular weight of the branched-block copolymer. Indeed, by having a number of arms n of at least 4, the degradable branched-block copolymer of the present invention can have a molecular weight sufficiently high for the intended applications - *i.e.* typically a molecular weight of at least 10 000 g/mol, even at least 20 000 g/mol - while having a polymer chain length, defined by the numbers m and I, short enough to provide a good reactivity and thus a high crosslinking yield.

Preferably, the polyether of the central core is selected from the group consisting of polyethylene glycol (PEG), poloxamer and poloxamine. Preferably, the polyether central core is a PEG central core.

Advantageously, each arm of the polyether central core is substituted with a degradable polymer chain, said degradable polymer chain being constituted by I unit(s) of a degradable polymer. The polymer chain may comprise one degradable polymer, or a mixture of at least two degradable polymers. The polymer chain may be for example a block copolymer or a polymer "ABABABABA".

Preferably, the degradable polymer of the degradable polymer chain is selected from the group consisting of a polyester, a polycarbonate and mixtures thereof. Advantageously, the degradable polymer chain is hydrophobic.

Advantageously, the degradable polymer of the degradable polymer chain is a polyester, for example selected from the group consisting of poly(lactide) (PLA), poly(ε-caprolactone) (PCL), polyhydroxybutyrate (PHB), polyhydroxybutyrate-co-hydroxyvalerate (PHBV), polyglycolic acid (PGA), poly(3-hydroxyvalerate), polydioxanone, Poly(trimethylene carbonate) (PTMC) and mixture thereof. Preferably, the degradable polyester is selected from the group consisting of poly(lactide) (PLA), poly(ε-caprolactone) (PCL), polyhydroxybutyrate (PHB), polyhydroxybutyrate-co-hydroxyvalerate (PHBV), polyglycolic acid (PGA), poly(3-hydroxyvalerate), polydioxanone, and mixture thereof. More preferably, it is PLA.

Typically, the branched-block copolymer has a molecular weight advantageously higher than 10 000 g/mol, preferably higher than 15 000 g/mol, more preferably higher than 20 000 g/mol, even more preferably higher than 25 000 g/mol.

In some embodiments, each polymer chain of the degradable branched-block copolymer is functionalized at its extremity with an aryl-azide derivative, such as an azidobenzoyl group, more preferably the 4-azidobenzoyl group (Bz-N₃).

The light activation, such as UV-light activation, of the aryl-azide function results in the formation of highly reactive nitrene species. Nitrene are carbene analogues wherein the nitrogen atom has an electrophile behaviour and is for example able to be inserted into CH bonds to form an amine bond.

In some embodiments, each polymer chain of the degradable branched-block copolymer is functionalized at its extremity with an olefinic group such as an allyl group, a maleimide group or a (meth)acrylate group, preferably with a (meth)acrylate group, more preferably with a methacrylate group.

The degradable branched-block copolymer may be regarded as water-soluble. Such water-solubility property results from the ratio between the hydrophilic polyether core and the preferably hydrophobic polymer chains. In the branched-block copolymer, said ratio is represented by the ratio m/l being strictly superior to 3 and preferably the I/m ratio is of 4 or more..

In these embodiments, the degradable polyester of the degradable polymer chain is preferably an amorphous PLA₅₀.

In a first variant, the core of the branched-block copolymer is linear, and thus different from a star-shaped or hyperbranched central core, resulting in a linear block-copolymer. In particular, the linear polyether central core is a polyethylene glycol (PEG), and/or poloxamer, preferably a PEG central core. Advantageously, the PEG central core consists in repeated units of the PEG monomer aligned one after the other and substituted by functional groups R on each side, each functional group R providing a single bond or 1 terminal function or atom from which the degradable polymer chain can extend. The terminal functions or atoms are preferably an oxygen atom or NH groups.

In this first variant, the branched-block copolymer with a linear central core can be represented by the following formula (III): 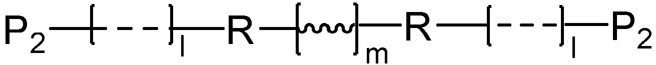 wherein 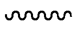 is the monomer unit which forms the hyperbranched polyether central core, and
R is a single bond or a functional group comprising one terminal function being an oxygen atom or a NH group, ,
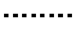 is the monomer unit of the degradable polymer constituting the degradable polymer chain,
P₂ is the photoreactive group and
m ranges from 4 to 600 and
I ranges from 2 to 400.

In the branched-block-copolymer with linear polyether central core, each terminal function is substituted by one degradable polymer chain as defined above. According to this variant, the branched-block-copolymer may be viewed as a linear triblock copolymer.

In a second variant, the core of the branched-block copolymer is hyperbranched, and thus different from a star-shaped or linear polyether central core, resulting in a hyperbranched block-copolymer.

In particular, the hyperbranched polyether central core is a polyethylene glycol (PEG), poloxamer and/or poloxamine, preferably a hyperbranched PEG central core.

Advantageously, the hyperbranched polyether central core may be a linear PEG consisting in repeated units of the PEG monomer aligned one after the other and substituted by functional groups R on each side, each functional group R providing n/2 terminal functions or atoms from which the degradable polymer chain can extend. The terminal functions or atoms are preferably an oxygen atom or NH groups. For example, such hyperbranched cores may be of the following formulas:

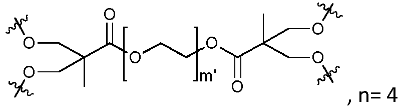

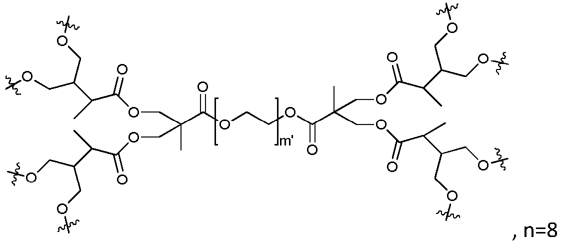

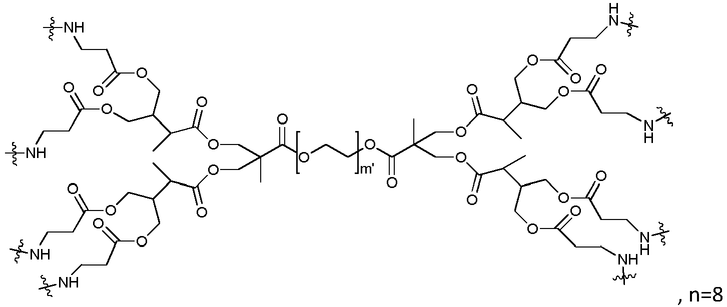

According to this second variant, the hyperbranched-block copolymer with a hyperbranched central core can be represented by the following formula (IV): 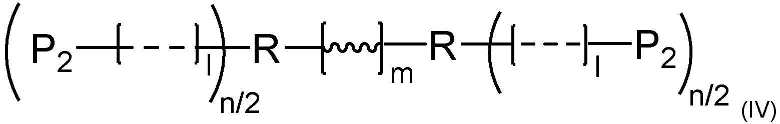 wherein
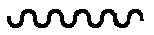 is the monomer unit which forms the hyperbranched polyether central core,
R is a multivalent branched functional group comprising a number n/2 of terminal functions or atoms, the terminal functions or atoms being an oxygen atom or a NH group, each of this terminal function being linked to one polymer chain,
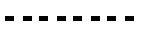 is the monomer unit of the degradable polymer constituting the degradable polymer chain,
P₂ is the photoreactive group and
n is an integer of at least 3, preferably at least 4,
m ranges from 4 to 600 and
I ranges from 2 to 400.

Preferably n is an integer of at least 4, advantageously ranging from 4 to 32, preferably from 4 to 16, more preferably n is equal to 4, 8 or 16.

In the branched-block copolymer with hyperbranched polyether central core, each terminal function is preferably an oxygen atom or a NH group and is substituted by one degradable polymer chain as defined above.

The branched-block copolymer with a linear (first variant) or hyperbranched (second variant) central core is advantageously symmetrical. In particular, n is an even integer, and all R functional groups are identical, each group R providing a number n/2 of identical terminal functions or atoms as defined above, each terminal function being substituted by the same polymer chain.

In a second variant, the degradable branched-block copolymer is a star-shaped copolymer, comprising a star-shaped polyether central core having n arms and polymer chains extending from each arm of the polyether central core, n being an integer of at least 3, preferably at least 4, each arm comprising m unit(s) of the monomer corresponding to the polyether core, and each polymer chain being constituted by I unit(s) of a degradable polymer, characterized in that each polymer chain is identical and functionalized at its extremity by a photo-reactive group selected from the group consisting of an azide group, a thiol group or an olefinic group (such as an allyl group, a maleimide or a (meth)acrylate group), and m is comprised between 4 and 400 and I is comprised between 4 and 1500.

The star-shaped copolymer may be represented by the following formula (II): 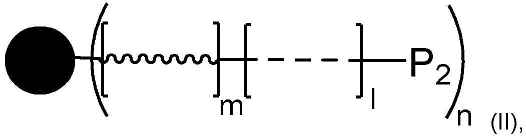 wherein 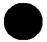 is the star polyether central core,
is the monomer unit corresponding to the polyether core, m being an integer from 4 to 400,
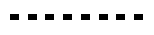 is the monomer unit of the degradable polymer constituting the polymer chain, I being an integer from 4 to 1500,and
P₂ is the photoreactive group defined above, and
n is an integer of at least 3, preferably at least 4, more preferably n ranges from 4 to 8, even more preferably n is 4, 6 or 8.

Advantageously, in this second variant, the number of arms n is of at least 4, and preferably ranges from 4 to 12, more preferably from 4 to 8. Advantageously, n is 4, 6 or 8, preferably 8. An 8-arm star-shaped block copolymer (corresponding to n = 8) is preferred in order to increase the number of reactive sites per molecule of copolymer.

In a preferred embodiment, the star-shaped polyether central core is a polyethylene glycol (PEG), poloxamer or poloxamine, preferably a PEG central core.

For example, a star-shaped (PEG)₄ₐᵣₘ compound responds to the formula:

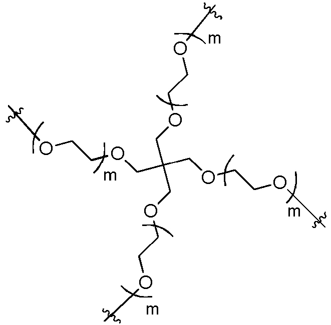

A star-shaped (PEG)₆ₐᵣₘ compound may be represented for instance by the formula :

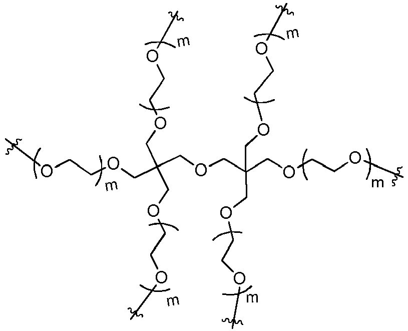

A star-shaped (PEG)₈ₐᵣₘ, for instance the 8-arm poly(ethyleneglycol) (tripentaerythritol), responds to the formula :

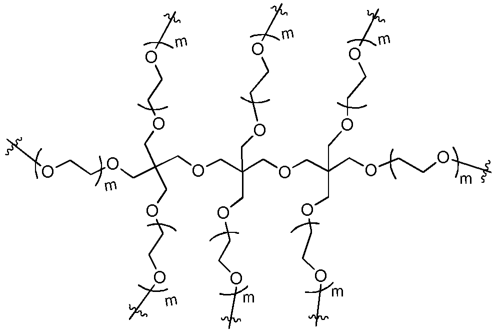

In some embodiments, each degradable polymer chain of the degradable star copolymer is functionalized at its extremity with an aryl-azide derivative, such as an azidobenzoyl group, more preferably the 4-azidobenzoyl group (Bz-N₃).

In some embodiments, the (aryl-azide)-functionalized degradable star copolymer responds to the following formula : 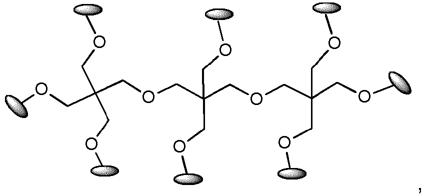 wherein 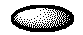 is the moiety [PEG-PLA-Bz-N₃] of formula: 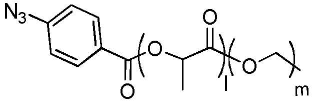 m and I being defined as above.

Such star block copolymer is also referred to as PEGs₈ₐᵣₘ10k-PLA₉₄-fN₃ or s-PLA-fN₃ in the following.

In some embodiments, each polymer chain of the degradable star copolymer is functionalized at its extremity with a (meth)acrylate group or a thiol group, preferably with a (meth)acrylate group, more preferably with a methacrylate group.

According to the present invention, the (meth)acrylate or thiol-functionalized degradable star copolymer has advantageously a number-average molecular weight lower than or equal to 100 000g/mol, preferably lower than or equal to 50 000 g/mol. A number-average molecular weight higher than 100 000 g/mol is often associated with a loss of reactivity of the copolymer due to a lower probability of the (meth)acrylate or thiol functions to react.

The number-average molecular weight Mₙ, as well as the dispersity $D = \frac{{M}_{w}}{{M}_{n}}$, wherein M_{w} represents the weight-average molecular weight, may be measured by size-exclusion chromatography (SEC). The number-average molecular weight Mₙ may also be measured by proton NMR (¹H NMR).

According to the present invention, the (meth)acrylate or thiol-functionalized degradable star copolymer is water-soluble. Such water-solubility property results from the ratio m/l, i.e. the ratio between the hydrophilic polyether core and the preferably hydrophobic polymer chains, said ratio m/l being strictly superior to 3, and preferably of 4 or more.

In preferred embodiments, the (meth)acrylate-functionalized degradable star block copolymer according to the present invention responds to the following formula: 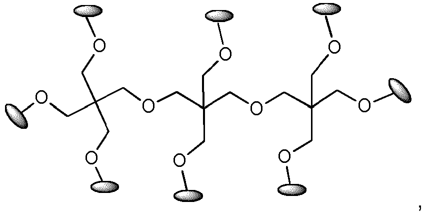 wherein 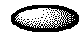 is the moiety [PEG-PLA-MA] of formula: 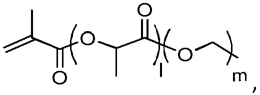 (also referred to as PEG₈ₐᵣₘ10k-PLA₅₀-MC or s-PLA-MA in the following), m being comprised between 4 and 400 and I being comprised between 4 and 1500, or to the following formula: 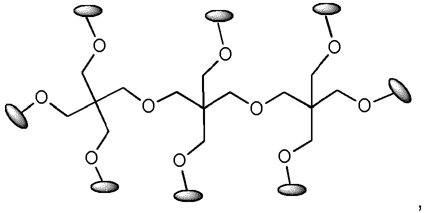 wherein 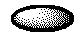 is the moiety [PEG-PLA-A] of formula: 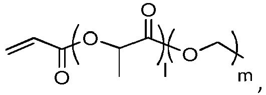 (also referred to as PEG₈ₐᵣₘ10k-PLA₅₀-A or s-PLA-A in the following), m being comprised between 4 and 400 and I being comprised between 4 and 1500.

### Photoreactive groups P1 and P2 adapted to react in a crosslinking reaction

### Advantageously:

- the photoreactive group P1 is an aryl-azide derivative, preferably an azidobenzoyl group such as 4-azidobenzoyl group, and the photoreactive group P2 is an olefinic group, such as an allyl group or a (meth)acrylate group, or
   the photoreactive group P1 is an olefinic group, such as an allyl group or a (meth)acrylate group, and the photoreactive group P2 is an aryl-azide derivative, preferably an azidobenzoyl group such as 4-azidobenzoyl group, or
- the photoreactive groups P1 and P2 are both independently an aryl-azide derivative, preferably an azidobenzoyl group such as 4-azidobenzoyl group, or
- the photoreactive groups P1 and P2 are both independently a (meth)acrylate group, or
- the photoreactive group P1 is an olefinic group, such as an allyl group, a maleimide group or a (meth)acrylate group, and the photoreactive group P2 is a thiol group, or
- the photoreactive group P1 is a thiol group and the photoreactive group P2 is an olefinic group, such as an allyl group, a maleimide group or a (meth)acrylate group.

In preferred embodiments, the photoreactive group P1 and P2 are both a (meth)acrylate group. In some embodiments wherein P1 or P2 is an aryl-azide derivative - preferably an azidobenzoyl group such as 4-azidobenzoyl group (Bz-N₃) - then the other photoreactive groupe (respectively P2 or P1) may be a single C-H bond. Preferably, in those embodiments, P2 is an aryl-azide derivative and P1 is a single C-H bond.

### Optional ingredients

In some embodiments, the hydrogel is further based on at least one active ingredient, in particular selected from the group consisting of antibiotics, healing agents, antimicrobial peptides, moisturizing agents, and mixtures thereof.

The active ingredient may be a medicinal or a non-medicinal plant extract. Preferably, the plant is selected from the group consisting of *Psiloxylon mauritianum, Pelargonium x graveolens, Dodonaea viscosa, Hypericum lanceolatum, Hubertia ambavilla, Aphloia theiformis, Syzygium cumini, Ayapana triplinervis,* or mixtures thereof.

The active ingredient advantageously includes a polyphenolic agent in glycosylated form or not, such as gallic acid or flavonoid hexoses.

Medicinal and non-medicinal plant extracts may be produced by (1) treating and drying plant raw material at temperatures ranging from 30 to 80°C for a period ranging from 1h to 24h (2) grinding plant material and soaking it in a mixture of solvents (acetone, water, ethanol) at different ratios (3) filtering the macerate to remove impurities.

### Combinations

In preferred embodiments, the degradable branched-block copolymer has a hyperbranched - in particular of formula (II) as defined herein - or star-shaped polyether central core, more preferably a star-shaped polyether central core of formula (III) as defined herein.

In these preferred embodiments, the hyperbranched or star-shaped polyether central core is advantageously a polyethylene glycol (PEG), poloxamer or poloxamine, preferably a PEG central core, and in formulae (II) and (III), n is of 4 or more, preferably n ranges from 4 to 12, more preferably from 4 to 8. Advantageously, n is 4, 6 or 8, preferably 8.

In these preferred embodiments, advantageously:
- the photoreactive group P1 is an aryl-azide derivative, preferably an azidobenzoyl group such as 4-azidobenzoyl group, and the photoreactive group P2 is an olefinic group, such as an allyl group or a (meth)acrylate group, or
   the photoreactive group P1 is an olefinic group, such as an allyl group or a (meth)acrylate group, and the photoreactive group P2 is an aryl-azide derivative, preferably an azidobenzoyl group such as 4-azidobenzoyl group, or
- the photoreactive groups P1 and P2 are both independently an aryl-azide derivative, preferably an azidobenzoyl group such as 4-azidobenzoyl group, or
- the photoreactive groups P1 and P2 are both independently a (meth)acrylate group, or
- the photoreactive group P1 is an olefinic group, such as an allyl group, a maleimide group or a (meth)acrylate group, and the photoreactive group P2 is a thiol group, or
- the photoreactive group P1 is a thiol group and the photoreactive group P2 is an olefinic group, such as an allyl group, a maleimide group or a (meth)acrylate group. Preferably, the photoreactive group P1 and P2 are both a (meth)acrylate group.

In these preferred embodiments, advantageously, the weight ratio between the gelatin compound a) and the degradable branched-block copolymer b) ranges from 5 to 95 %, preferably from 25 to 75%.

### III. Method for preparing a biomaterial

The invention further provides a method for preparing a biomaterial, said method comprising: mixing the dehydrated hydrogel of the invention or obtained by the method of the invention, with an aqueous solution optionally comprising at least one active ingredient, and mixtures thereof, to obtain a rehydrated hydrogel ready to be applied on a wound to be treated, typically a chronic wound, such as bedsore, leg ulcer or diabetic ulcer.

The aqueous solution comprises a solvent including at least 80 wt%, preferably at least 90 wt%, of water. In some embodiments, the aqueous solution comprises a pharmaceutically acceptable co-solvent such as a pharmaceutically acceptable lower alcohol, preferably ethanol. In other embodiments, the solvent of the aqueous solution consists of water. Advantageously, the pH of the aqueous composition ranges from 4.5 to 8.5, preferentially from 5 to 7.5, advantageously from 6 to 7. In particular, the aqueous composition may be buffered, *i.e.* it may also comprise a buffer chosen from common buffers for the pH range 5 to 8, preferentially selected from the group consisting of acetate, lactate, tartrate, malate, maleate, succinate, ascorbate, carbonate, phosphate, Tris (Tris(hydroxymethyl)aminomethane), HEPES (2-[4-(2-hydroxyethyl)-1-piperazine]ethanesulfonic acid), MES (2-morpholinoethanesulfonic acid) and mixtures thereof, preferentially acetate, phosphate, Tris, lactate, tartrate, carbonate; MES or mixtures thereof, more preferably the phosphate or acetate buffered solutions.

The aqueous composition may further comprise additives such as antioxidants, salts, and/or stabilizers. Exemplary formulations may be found in the general literature, including Remington's for Pharmaceutical Science 18th Edition (1990), Mack. Pub. For instance, sterile aqueous or saline solutions can be prepared with galenic adjuvants (lactose, methylcellulose, mannitol), and/or surfactants (lecithins, Tween^{®} or similar products).

Preferred salts include sodium chloride.

Optionally, the aqueous solution may comprise at least one active ingredient, in particular as defined below.

The at least one active ingredient is in particular selected from the group consisting of antibiotics, healing agents, antimicrobial peptides, moisturizing agents, and mixtures thereof. Preferably, the active ingredient is a medicinal and/or non-medicinal plant extract. The active ingredient advantageously includes a polyphenolic agent in glycosylated form or not, such as gallic acid or flavonoid hexoses.

Medicinal and non-medicinal plant extracts may be produced by (1) treating and drying plant raw material at temperatures ranging from 30 to 80°C for a period ranging from 1h to 24h (2) grinding plant material and soaking it in a mixture of solvents (acetone, water, ethanol) at different ratios (3) filtering the macerate to remove impurities.

In embodiments wherein the dehydrated hydrogel already contains one or more active ingredient(s), the aqueous solution is preferably free of optional active ingredients.

However, preparing a biomaterial using a dehydrated hydrogel free of optional active ingredients, and rehydrated with an aqueous solution comprising at least one active ingredient is generally preferred as it allows including in the aqueous solution sensitive active ingredients, which would for instance degrade and/or react during crosslinking, or degrade upon freeze-drying or storing.

### IV. Biomaterial and uses thereof

The invention further provides a biomaterial comprising a rehydrated hydrogel advantageously ready to be applied on a wound to be treated, wherein the rehydrated hydrogel is obtainable or obtained by the method of section III.

The wound to be treated is typically a chronic wound, such as bedsore, leg ulcer or diabetic ulcer.

In all embodiments, the biomaterial provides a physical protection to the flesh in the wound, of the flesh in the wound, thus avoiding infections and resulting in a faster and/or better recovery. Advantageously, in embodiments wherein the biomaterial includes at least one active ingredient, the biomaterial further allows to release the at least one active ingredient in the wound, improving even more the recovery process.

The invention thus further provides a use of the biomaterial of the invention as wound dressing. The invention further provides a biomaterial of the invention for use for skin regeneration or for treating a wound, in particular a chronic wound, such as a bedsore, an ulcer or a diabetic wound.

### DRAWINGS

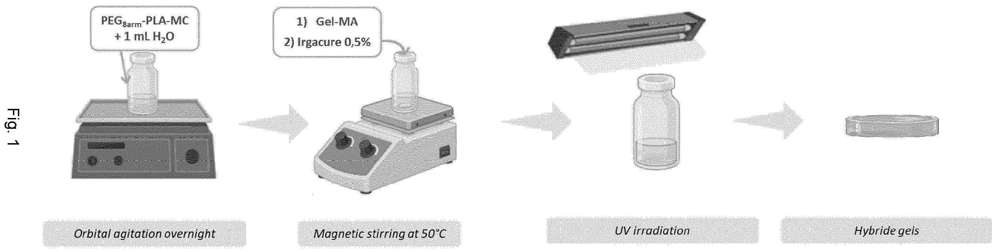

Figure 1 : Scheme illustrating an embodiment of the method of preparing a dehydrated hydrogel, used in example 3.
Figure 2 : Picture of the cured hydrogels of example 3.2, after 5 minutes UV irradiation (left column) and 10 minutes UV irradiation (right column). a = cured hydrogel obtained from composition 1; b = cured hydrogel obtained from composition 2; c = cured hydrogel obtained from composition 3; d = cured hydrogel obtained from composition 4.
Figure 3 : SEB image of the dehydrated hydrogels of example 3.3. a = dehydrated hydrogel obtained from composition 1; b = dehydrated hydrogel obtained from composition 2; c = dehydrated hydrogel obtained from composition 3; d = dehydrated hydrogel obtained from composition 4.Upper line: picture of dehydrated hydrogels surface. Bottom line: picture of dehydrated hydrogels surface.

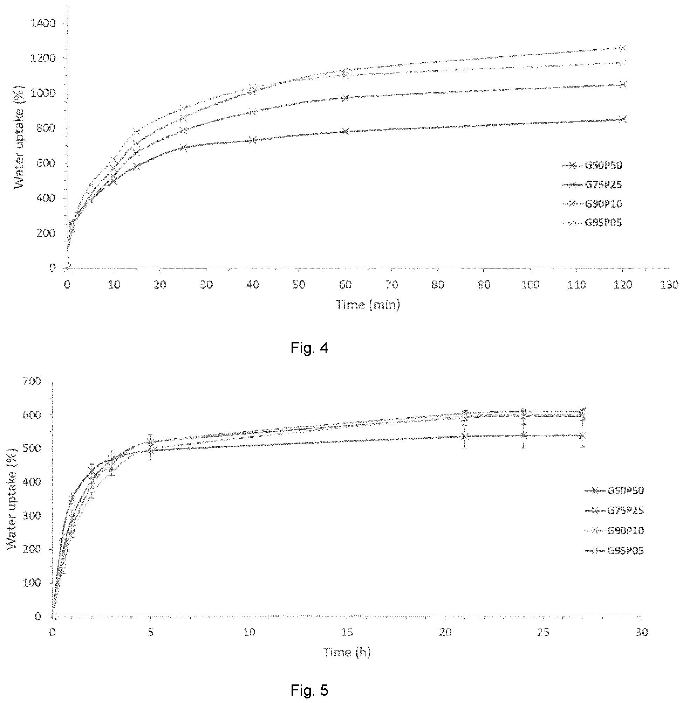

Figure 4 : Swelling capacity as a function of time, of the dehydrated hydrogels of example 3.3. Ordinates = water content in wt%; abscissa = time in minutes.
Figure 5 : Swelling capacity as a function of time, of the cured hydrogels of example 3.2. Ordinates = water content in wt%; abscissa = time in minutes.

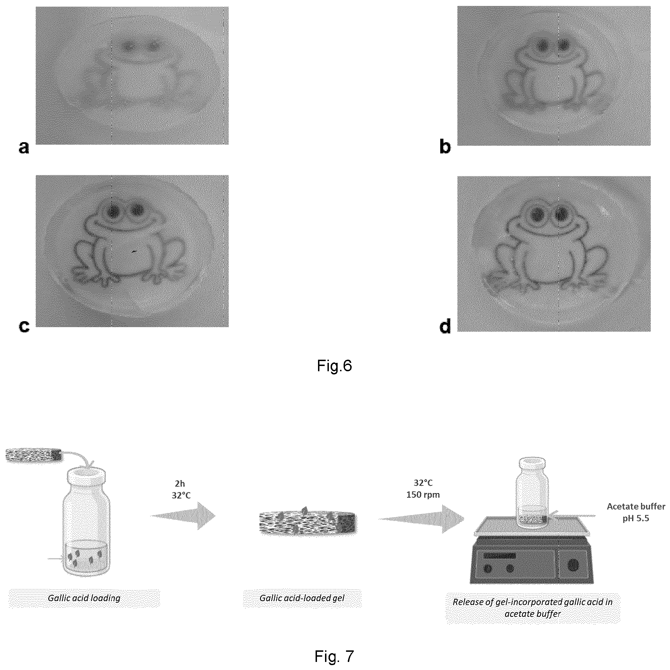

Figure 6 : Pictures of the opcity test conducted using the rehydrated hydrogels of example 4. a = rehydrated hydrogel obtained from composition 1; b = rehydrated hydrogel obtained from composition 2; c = rehydrated hydrogel obtained from composition 3; d = rehydrated hydrogel obtained from composition 4.
Figure 7 : Scheme depicting the protocol used in example 5.1 for preparing a biomaterial according to a particular embodiment of the method of the invention, and the protocol of the release test of example 5.2.

### EXAMPLES

The following examples are given for illustrative purposes only and should not be understood as limiting the invention in any way.

### Methods

### Measure of the functionalization rate (FR)

The functionalization rate (FR) is measured with a colorimetric method using 2,4,6-trinitrobenzene sulfonic acid (TNBS) as the indicator. The TNBS indeed forms an orange compound when reacting with amines.

In particular, when measuring the FR of the (functionalized) gelatine compound, the TNBS reacts with the free amines of the lysine residues of the gelatine.

### Example 1: Preparation of a gelatin compound functionalized with a methacrylate group (Gel-MA)

### 1.1. Preparation of sample Gel-MA 1

A first sample called Gel-MA1 was prepared Following the method described by Shirahama et al. (Synthesis. Sci Rep. 2016;6(1):31036):
5 g of Gelatine (Gel) were solubilized in 50 mL of an aqueous solution buffered using a carbonate-bicarbonate (CB) buffer (pH = 9), at 50°C. Once the solution is homogenous, the pH is once more adjusted to pH = 9 using NaOH (5 M) and/or HCl (6 M) solutions. Methacrylic anhydride (MAA) is then added, and the reaction medium is left to stir for 3h. The solution is then dialyzed in water for 72 hours at 50°C and finally freeze-dried to yield a solid Gel-MA1. Table 1 summarizes the reaction conditions:

**Table 1**

| | |
|---|---|
| Buffer Molarity (M) | 1 |
| Gelatine (% w/v) | 10 |
| Molar Ratio MAA/amine | 2,485 |
| MAA (mmol) | 3,55 |
| MAA (mL) | 0,498 |

The functionalization rate (FR) of Gel-MA1 was determined to be 79,36%, using the colorimetric method described above.

### 1.2. Preparation of sample Gel-MA2

A second sample called Gel-MA2 was prepared using the same protocol as Gel-MA1, but this time adjusting the pH of the reaction to 9 every 30 minutes, to optimize the reactivity of the amine functions, as described in Lee et al. (RSC Adv. 2015;5(128):106094-7).

The functionalization rate (FR) of Gel-MA1 was determined to be 85,52%, using the colorimetric method described above.

### 1.3. Variation of the Molar Ratio MAA/amine

Other samples were prepared using the same protocol as Gel-MA1, but varying the molar Ratio MAA/amine. The reaction conditions and functionalization rates (FR) are summarized in table 2.

**Table 2**

| Reaction Conditions | **Gel-MA3** | **Gel-MA4** | **Gel-MA5** |
|---|---|---|---|
| Molar Ratio MAA/amine | 1,836 | 2,080 | 2,080 |
| MAA (mmol) | 2,62 | 2,97 | 2,97 |
| MAA (mL) | 0,368 | 0,416 | 0,416 |
| Functionalization rate (FR) | **76,36** ± 3,56 | **66,17** ± 8,47 | **65,28** ± 2,28 |

### Example 2: Preparation of a degradable branched-block copolymer functionalized with a methacrylate group (PEG₈ₐᵣₘ-PLA-MA)

### 2.1. Preparation of the PEG₈ₐᵣₘ-PLA polymer, with Mn = 27 500 g/mol

20 g of a 8-arm star-shaped PEG polymer with Mₙ = 20 000 g/mol noted PEG₈ₐᵣₘ20k, used as the polymerization initiator, and 9,2 g of D,L-lactide are solubilized in 100 mL anhydrous toluene under stirring and under argon for about 1h. Then, 0,33 g of Tin(ll) 2-ethylhexanoate, the polymerization catalyst, is added. The reaction mixture is then stirred under argon at 110°C for 24h. It is then concentrated under reduced pressure and precipitated with diethyl ether. The resulting precipitate is then dried.

The obtained polymer was solubilized in THF for the SEC analysis. The polymers dispersity, evaluated by SEC in THF was 1.13.

The number-average molecular weight Mₙ of the PEG₈ₐᵣₘ-PLA is calculated from H-NMR and was find to be ${M}_{n} \left({PEG}_{8 arm}-PLA\right) = 27 375 g / mol .$

### 2.2 Functionalization of the PEG₈ₐᵣₘ-PLA polymer with methacryloyl chloride (MC)

The PEG₈ₐᵣᵣ-PLA copolymer obtained in 2.1 is solubilized in dichloromethane (10% w/v). Triethylamine is then added (5 eq/OH). The reaction medium is then cooled to 0 °C for about 1h under stirring, before dropwise addition of methacryloyl chloride (5 eq/OH), and further cooling at 0°C for another 1h. The reaction medium is then heated under stirring to 45°C for 72h. It is then filtered and washed. The organic phase is recovered and concentrated under reduced pressure and precipitated using diethyl ether. The resulting precipitate is then dried. The dispersity of the PEG₈ₐᵣₘ-PLA-MA was 1.09 as measured by SEC-THF.

The number-average molecular weight Mn of the functionalized PEG₈ₐᵣₘ-PLA copolymer is calculated using H-NMR and find to be : $27 938 g / mol$

The calculated Mn(PEG₈ₐᵣₘ-PLA-MA) is comparable to that of the starting PEG₈ₐᵣₘ-PLA, thus demonstrating that the functionalization step did notdegrade the branched block-copolymer. The NMR spectrum further allows to determine the functionalization rate FR.

To calculate the FR, the theoretic signal obtained for a 100% functionalized polymer is first evaluated following equation (8). The FR is then calculated as the ratio of the sum of the integrated peaks attributed to the methacrylates MA signals at 5,6 ppm and 6,2 ppm, to the theoretical signal of the 100% functionalized polymer, following equation (9). $Signal 100 % MA = 8 \times 2 \times Signal 1 H EG$ $RF = \frac{\sum \left(Integrales of methacrylate peaks\right)}{Signal 100 % MA} \times 100$

The calculated FR is of about 100%.

### Example 3: Preparation of a dehydrated PEG₈ₐᵣₘ-PLA-MA / Gel-MA hydrogel

### 3.1. Forming an aqueous composition comprising the Gel-MA and the degradable branched-block copolymer PEG₈ₐᵣₘ-PLA-MA

The PEG₈ₐᵣₘ-PLA-MA (introduced according to the required m/v percentage) and the photoinitiator (Irgacure 2959, 0.5 % by weight of material related to the weight of PEG₈ₐᵣₘ-PLA-MA + Gel-MA) are solubilized in water (1 mL) under stirring using an orbital shaker. Then Gel-MA4 or Gel-MA5 is added to the solution, and the resulting reaction medium is heated to 50°C under magnetic stirring.

Different compositions were obtained, using the Gel-MA/PEG₈ₐᵣₘ-PLA-MA ratios indicated in table 3.

**Table 3. "Comp." stands for composition.**

| Name of composition | Comp. 1 | Comp. 2 | Comp. 3 | Comp. 4 |
|---|---|---|---|---|
| Gel-MA/PEG₈ₐᵣₘ-PLA-MA mass ratio | 50/50 | 75/25 | 90/10 | 95/5 |
| PEG₈ₐᵣₘ-PLA-MA (in g) | 0.5 | 0.25 | 0.1 | 0.05 |
| Gel-MA (in g) | 0.5 | 0.75 | 0.9 | 0.95 |
| Irgacure 2959 (in mg) | 5 | 5 | 5 | 5 |

### 3.2 Crosslinking step

The aqueous composition obtained in 3.1 is then irradiated under UV light (between 320-390 nm) at 52 mW/cm² during 5 or 10 min.

The use of an initiator or not, the photoinitiator content as well as the irradiation time were optimized in preliminary tests using Gel-MA alone or PEG₈ₐᵣₘ-PLA-MA alone.

**Table 3. "Comp." stands for composition.**

| Name of composition | Comp. 1 | Comp. 2 | Comp. 3 | Comp. 4 |
|---|---|---|---|---|
| Gel-MA/PEG₈ₐᵣₘ-PLA-MA ratio | 50/50 | 75/25 | 90/10 | 95/5 |
| Gel Fraction after 5 min irradiation | 91.63 | 95.35 | 97.28 | 92.86 |
| Gel Fraction after 10 min irradiation | 90.26 | 95.44 | 96.60 | 98.17 |

All tested ratios allow the formation of cured hydrogels with gel fractions of more than 90%. Also, for a given ratio, there is no significant difference in crosslinking efficiency at 5 and 10 minutes irradiation times. However, for different ratios, it was observed that the more the Gel-MA proportion, the greater the percentage of gel fraction.

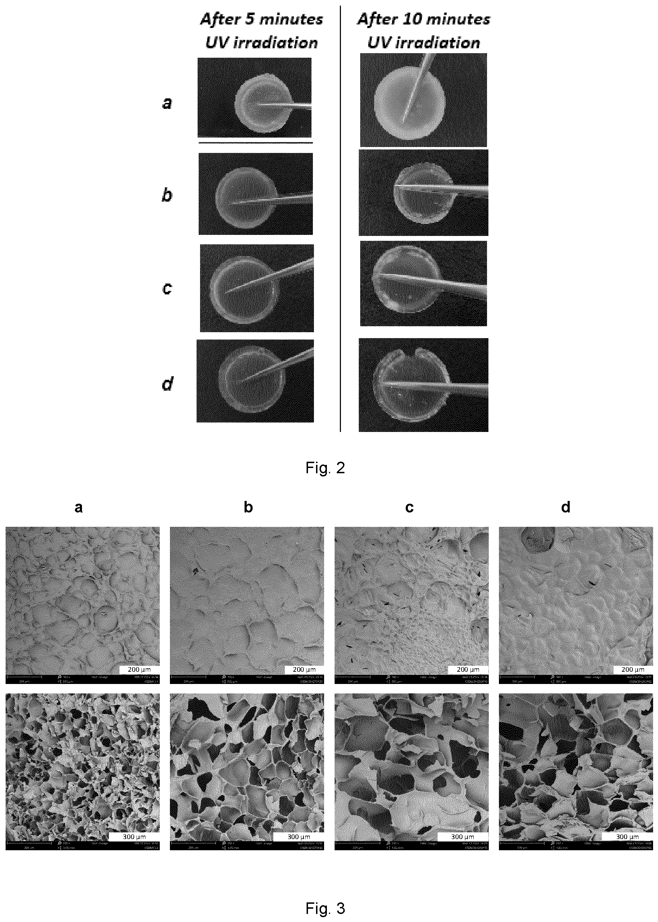

After 24 hours of immersion in water at 55°C, all cured hydrogels appeared to be homogenous homogeneous (see Fig. 2).

### 3.3 Freeze-drying step

The cured hydrogels are placed in a freezer at -20°C and left to stand there overnight.

The freezed cured hydrogels are then freeze-dried (transport from the freezer to the freeze drier is carried out under cooling with liquid nitrogen to avoid undesired liquefaction) in a freeze-drier ALPHA 1-4 LDplus by CHRIST, at -50°C and 0.03 mBar.

### Example 4. Rehydration - preparation of a biomaterial

The dehydrated hydrogels prepared in example 3 were weighed and then immersed in water at 32°C.

At different times tx, the rehydrated hydrogels are removed from the water and placed on absorbent paper in order to absorb excess water on the surface. The water content of the hydrogels is determined following equation (9), with masseₜ₀, the mass of the dry gel before immersion in water and masseₜₓ, the mass of the gel at time x (*i.e.* after a time x d immersion in water). $Water content at tx \left(%\right) = \frac{{mass}_{{t}_{x}} - {mass}_{{t}_{0}}}{{mass}_{{t}_{0}}} \times 100$

The results obtained show that the maximum swelling capacities are between 850% and 1250% depending on the hydrogel composition, after 2 hours of immersion in water.

Comparative tests using non-dehydrated cured hydrogels give significantly lower maximum swelling capacities of between 500% and 600%, and these are obtained after an immersion time of 16h, i.e. 8 times longer than the immersion times of the dehydrated hydrogels. These differences in time for reaching maximum swelling capacity can be explained by the presence of pores in the freeze-dried hydrogels which allow water to penetrate the network more quickly.

Opacity tests were performed by depositing the rehydrated hydrogels on a simple image (here a frog) and observing whether the drawing is still recognizable through the rehydrated hydrogel. All hydrogels have a satisfactory transparency, including the rehydrated hydrogel obtained from composition 1 which, unlike the others, is only translucent. Thus, all the hydrogels obtained have an acceptable opacity for the desired applications

### Example 5. Rehydration - preparation of a biomaterial including an active ingredient - gallic acid

### 5.1. Rehydration of the dehydrated hydrogel with a gallic acid solution

Gallic acid (hereinafter "GA") was used as a model active ingredient, as it is a phenolic compound with antimicrobial activity.

Different quantities of gallic acid were therefore loaded into freeze-dried G50P50 gels and their release was studied. Loading was carried out by total immersion of the dry gel in a GA solution of known concentration. For this purpose, the loading volume was defined from the maximum swelling capacity determined for each condition as indicated in equation (10). ${mass}_{after swelling} = {mass}_{dry gels} \times \left(1+\frac{swelling ratio}{100}\right)$ and ${mass}_{of loaded water} = {mass}_{after swelling} - {mass}_{dry gels}$

Amounts of 0.05 mg; 0.1 mg and 1 mg of GA were loaded into G50P50 gels. The swelling ratio of G50P50 reached after 2h of immersion in water is 849.50% and the average mass of these dry gels (n=3) is 90,1 mg so it is deduced that : $Loading volume = {mass}_{of loaded water} = 0,765 mL$

The gels were immersed in 5 mL solutions of GA dissolved in water at concentrations of 0.0653 mg/mL, 0.131 mg/mL and 1.31 mg/mL to load 0.05 mg, 0.1 mg and 1 mg respectively into the gels. These loads were carried out under conditions where maximum swelling capacity was reached, i.e. at 32°C for 2h.

### 5.2. Release kinetics of the gallic acid

The GA was released in an acetate buffer solution at pH 5.47, since the pH of a normal wound is between 5.5 and 6.5 and polyphenols are stable at acidic pH values, particularly between 4 and 6.

In pill containers, loaded and unloaded G50P50 gels (n=3/condition) were immersed in 5 mL of acetate buffer solution under orbital shaking at approximately 140 rpm and at 32°C, corresponding to skin temperature. Uncharged gels were studied in order to assess the absorbance of their possible degradation, which will be deduced from samples taken from gels charged with GA.

At each time interval (30 min, 1h, 3h, 6h, 1D, 3D, 5D), take half of the buffer volume present in the pill containers for the analyses and add the same volume of fresh acetate buffer. The samples taken were then analysed using HPLC to detect the amount of GA released. To do this, samples were filtered through RC filters (0.45 µm) and injected at 10 µL into the Kinetex 2.6 µm C-18 100 A column (100 x 4.6 mm). The contents of the column were eluted with a gradient mixture of water (at 1 ‰ TFA) (A) and acetonitrile (at 1 ‰ TFA) (B) at a flow rate of 0.8 mL/min, with 10% B at 0-2 min, 10-35% B at 2-6 min, 35-10% B at 6-19.9 min. The column temperature was maintained at 40°C and the detection wavelength was set at 280 nm.

The results obtained for the study of GA release kinetics show that a 'burst release' phenomenon is common to all the loaded quantities studied. In fact, for the 0.05 mg and 0.1 mg quantities, between 30 and 40% of the GA was released after 3h before reaching a plateau indicating that there was no further release, so the unreleased part of the GA would have interacted with the gels. As for the 1 mg, 100% of the GA was released after 5 hours.

## Claims

1. A dehydrated hydrogel based on:
a) a gelatin compound consisting of a gelatin functionalized with at least one photoreactive group P1 typically selected from the group consisting of an aryl-azide group, a thiol group, and aan olefinic group such as an allyl group, a maleimide group or a (meth)acrylate group, and
b) a degradable branched-block copolymer,
the degradable branched-block copolymer comprising a polyether central core having n arms and degradable polymer chains extending from each arm of the polyether central core, the polyether central core being star-shaped or linear, each degradable polymer chain consisting of I monomer unit(s) of a degradable polymer,
wherein each degradable polymer chain is identical and functionalized at its extremity with a photoreactive group P2 adapted to react in a crosslinking reaction with the photoreactive group P1, the photoreactive group P2 being preferably selected from the group consisting of an aryl-azide group, a thiol group and an olefinic group such as an allyl group or a (meth)acrylate group,
wherein said degradable branched-block copolymer is of the following formula (I): 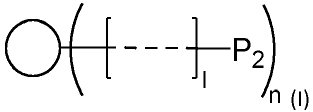
wherein 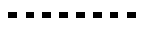 is the monomer unit of the degradable polymer constituting the degradable polymer chain,
P₂ is the photoreactive group defined above,
n is an integer of at least 2, and
the polyether central core 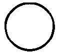
is linear, hyperbranched or star-shaped,
when the polyether central core is linear, 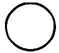
is 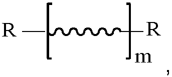
wherein R is linked to one polymer chain through a single bond or a functional group comprising a terminal function or atom selected among oxygen atom or NH group, and
when the polyether central core is hyperbranched, 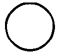
is 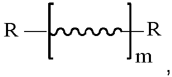
wherein R is a multivalent branched functional group comprising a number n/2 of terminal functions or atoms selected among oxygen atom or NH group, each of this terminal function being linked to one polymer chain and
when the polyether central core is star-shaped, 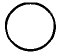
is 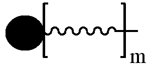
wherein 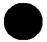 is the star polyether central core, 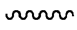
is the monomer unit corresponding to the polyether core, and
m ranges from 4 to 400 and I ranges from 4 to 1500..

2. The dehydrated hydrogel of claim 1, wherein the polyether of the central core of the degradable branched-block copolymer is a polyethylene glycol (PEG), a poloxamer or a poloxamine.

3. The dehydrated hydrogel of claim 1 or 2, wherein the degradable branched-block copolymer has a star-shaped polyether central core and is of the following formula (II): 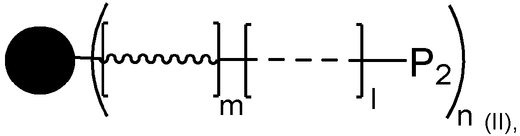 wherein 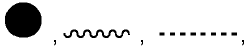 P₂, m and I are as defined in claim 1 or 2, and n is an integer of at least 3, preferably of at least 4, more preferably n ranges from 4 to 8, even more preferably n is 4, 6 or 8.

4. The dehydrated hydrogel of any of claims 1 to 3, wherein the degradable polymer of the degradable polymer chains of the degradable branched-block copolymer is selected from the group consisting of a polyester, a polycarbonate, and mixtures thereof, preferably a polyester.

5. The dehydrated hydrogel of claim 4, wherein the polyester is selected from the group consisting of poly(lactide) (PLA), poly(ε-caprolactone) (PCL), polyhydroxybutyrate (PHB), polyhydroxybutyrate-co-hydroxyvalerate (PHBV), polyglycolic acid (PGA), poly(3-hydroxyvalerate), polydioxanone, Poly(trimethylene carbonate) (PTMC) and mixtures thereof, preferably the polyester is PLA.

6. The dehydrated hydrogel of any of claims 1 to 5, wherein:
• the photoreactive group P1 is an aryl-azide derivative, preferably an azidobenzoyl group such as 4-azidobenzoyl group, and the photoreactive group P2 is an olefinic group, such as an allyl group or a (meth)acrylate group, or
the photoreactive group P1 is an olefinic group, such as an allyl group or a (meth)acrylate group, and the photoreactive group P2 is an aryl-azide derivative, preferably an azidobenzoyl group such as 4-azidobenzoyl group, or
• the photoreactive groups P1 and P2 are both independently an aryl-azide derivative, preferably an azidobenzoyl group such as 4-azidobenzoyl group, or
• the photoreactive groups P1 and P2 are both independently a (meth)acrylate group, or
• the photoreactive group P1 is an olefinic group, such as an allyl group, a maleimide group or a (meth)acrylate group, and the photoreactive group P2 is a thiol group, or
• the photoreactive group P1 is a thiol group and the photoreactive group P2 is an olefinic group, such as an allyl group, a maleimide group or a (meth)acrylate group.

7. The dehydrated hydrogel of any of claims 1 to 6, wherein the weight ratio between the gelatin compound and the degradable branched-block copolymer ranges from 5 to 95 %, preferably from 25 to 75%.

8. A method for preparing the dehydrated hydrogel of any of claims 1 to 7, comprising:
1) Forming an aqueous composition comprising the gelatin compound as defined in any of claims 1 to 7, and the degradable branched-block copolymer as defined in any of claims 1 to 7,
2) crosslinking the aqueous composition of step 1), to obtain a cured hydrogel and
3) freeze-drying the cured hydrogel of step 2).

9. The method of claim 8, wherein the step of forming an aqueous composition comprises heating at a temperature ranging from 15°C to 80°C, preferably from 20°C to 60°C, more preferably from 35°c to 55°C.

10. The method of claim 8 or 9, wherein the crosslinking step 2) is performed under photoreactive conditions, preferably under UV irradiation, and the aqueous composition of step 1) preferably further comprises an initiator, advantageously a photoinitiator.

11. A method for preparing a biomaterial, said method comprising:
mixing the dehydrated hydrogel of any of claims 1 to 7 or obtained by the method of any of claims 8 to 10, with an aqueous solution, to obtain a rehydrated hydrogel ready to be applied on a wound to be treated, typically a chronic wound, such as bedsore, leg ulcer or diabetic ulcer.

12. **The** method of claim 11, wherein the aqueous solution comprises at least one active ingredient, in particular selected from the group consisting of antibiotics, healing agents, moisturizing agents, and mixtures thereof.

13. Biomaterial comprising a rehydrated hydrogel advantageously ready to be applied on a wound to be treated, wherein the rehydrated hydrogel is obtainable or obtained by the method of claim 12 or 13.

14. Use of the biomaterial of claim 13 as wound dressing.

15. **The** biomaterial of claim 13, for use for skin regeneration or for treating a wound, in particular a chronic wound, such as a bedsore, an ulcer or a diabetic wound.
